# EUROPEAN PATENT APPLICATION

(11) **EP 4 645 335 A1**
(43) Date of publication of application: **05.11.2025**
(21) Application number: 25169556.5
(22) Date of filing: 09.04.2025
(51) Int. Cl.: G16H 40/67, G16H 50/20

(54) **SYSTEMS AND METHODS FOR AI-POWERED PATIENT MONITORING**

(30) Priority: 03.05.2024 US 202418655067
(71) Applicant: GE Precision Healthcare LLC, Waukesha, WI 53188 (US)
(72) Inventor: AHMED, Zuber, 560066 Bengaluru (IN)
(74) Representative: Kilburn & Strode LLP

(57) **Abstract**

Methods and systems are proposed that integrate real-time data analysis, adaptive alert thresholds, multimodal caregiver feedback, and data labeling to continuously refine alert generation models (500, 600, 650) relied on by patient monitoring systems (220). The proposed approach enhances the effectiveness of AI-powered patient monitoring systems (220) by addressing the challenges of inaccurate data labeling and high false alert rates. To minimize false alerts, caregivers are provided with an easy-to-use feedback tool (700) to confirm receipt of alerts, categorize alerts as true or false positives, and provide contextual information. This caregiver-provided information is then analyzed, and criteria may be extracted from the information that may be used to retrain or refine the alert generation models (500, 600, 650).

## Description

### FIELD

Embodiments of the subject matter disclosed herein relate generally to patient monitoring systems, and in particular, to alerts generated by patient monitoring systems.

### BACKGROUND

Monitoring physiological patient data of a patient is an important part of patient care, and physicians often desire to continuously monitor multiple physiological patient data of their patients in real time, such as blood pressure, oxygen saturation (SpO2), features of the electrocardiogram (ECG), and others. Patient monitoring often involves the use of several sensing devices to perform multiple physiological monitoring modalities, such as a pulse oximeter, a blood pressure monitor, a heart monitor, a temperature monitor, etc. Many patient monitoring devices offer multi-modality patient monitoring, where multiple different sensing devices for sensing different physiological patient data can be connected to a single patient monitor that is configured to collect, process, and/or display physiological information describing the patient's health condition. The patient monitor may also be connected to a wireless network accessible in the hospital environment, such that the multiple different sensing devices may communicate with the patient monitor wirelessly. A caregiver may monitor real-time physiological patient data of the patient remotely by viewing data of the patient monitor via a remote viewing application (e.g., on a smart phone), as the patient or the caregiver move around a hospital environment.

Additionally, a patient monitoring system may be configured to notify the caregiver in the event that a measurement of the physiological patient data exceeds a threshold value. For example, if a heart rate of the patient exceeds a threshold heart rate, an alert may be generated on the patient monitor and/or on a wireless device of the caregiver. However, current patient monitoring systems often struggle with inaccurate alerts due to pre-defined static thresholds and limited learning capabilities. This can lead to both missed events (false negatives) and unnecessary alerts (false positives), reducing caregiver trust and increasing workload.

### BRIEF DESCRIPTION

In one example, the problems described above are addressed by a method for a patient monitoring system, comprising receiving time-series patient data in real time from a patient monitor coupled to a patient; in response to an artificial intelligence (AI) model of the patient monitoring system detecting a deviation in the time-series patient data from expected time-series patient data, displaying an alert on the patient monitor, the alert including a graphical user interface (GUI) for receiving a feedback record regarding the alert from a user of the patient monitoring system, receiving a plurality of feedback records regarding alerts generated for a respective plurality of patients, via the GUI; and in response to the plurality of feedback records exceeding a threshold number of records, for each feedback record of the plurality of feedback records including a false alert, generating a standardized, predefined description of the false alert. In response to a number of feedback records having a same standardized, predefined description being greater than a threshold number, the method includes identifying a first set of patients having the same standardized, predefined description; identifying a second set of patients in an electronic medical record (EMR) database that have similar patient data and/or characteristics as the first set of patients, using a clustering model, the second set of patients including the first set of patients; updating the AI model based on patient data of the first set of patients; and using the updated AI model to generate alerts for the second set of patients.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 illustrates an exemplary patient monitoring environment, according to one or more embodiments of the present disclosure;
FIG. 2 is a schematic diagram showing an exemplary patient monitoring system, which may automatically generate patient status alerts for a caregiver, according to one or more embodiments of the present disclosure;
FIG. 3 is a flowchart that illustrates an exemplary method for generating the patient status alerts and collecting caregiver feedback on the alerts, according to one or more embodiments of the present disclosure;
FIG. 4A is a flowchart that illustrates an exemplary method for refining an AI model for predicting when to generate a patient status alert, based on caregiver feedback, according to one or more embodiments of the present disclosure;
FIG. 4B is a continuation of the flowchart of FIG. 4A, according to one or more embodiments of the present disclosure;
FIG. 5 shows a first exemplary rules-based model for determining when to generate a patient status alert, according to one or more embodiments of the present disclosure;
FIG. 6A shows a second exemplary rules-based model for determining when to generate a patient status alert, according to one or more embodiments of the present disclosure;
FIG. 6B shows a third exemplary rules-based model for determining when to generate a patient status alert, according to one or more embodiments of the present disclosure;
FIG. 7 shows a first exemplary patient status alert displayed by the patient monitoring system, according to one or more embodiments of the present disclosure;
FIG. 8 shows a list of codes for providing feedback with respect to a patient status alert, according to one or more embodiments of the present disclosure; and
FIG. 9 shows exemplary record of caregiver feedback stored in a memory of a patient monitoring system, according to one or more embodiments of the present disclosure.

### DETAILED DESCRIPTION

The following description relates to systems and methods for quickly and efficiently monitoring a status of patients in care units of a hospital or healthcare organization. Efficient patient management may include stabilizing patients with serious medical conditions and providing initial treatment, and once the patient is stable, diagnosing the patient and establishing a treatment strategy for the patient. Thus, efficient patient management may depend on an efficient patient monitoring process for regularly assessing a status of a plurality of patients.

Patient monitoring may include a number of different physiological monitoring devices, sensors, etc. capable of monitoring cardiac, respiratory, neurologic, hemodynamic, pulse oximetry, etc. parameters such as but not limited to electrocardiography (ECG), peripheral capillary oxygen saturation (SpO2), respiration rate, temperature, blood pressures, Entropy, blood glucose, and carbon dioxide. Patient monitoring is performed by way of many different forms and approaches with respect to data capture and communication technologies (e.g., hard-wired and wireless networking) and may include monitoring a patient locally (e.g., in-room wired or tethered to a monitor) and/or wirelessly (e.g., in-room, while in transport, ambulating telemetry). In addition to moveable roll stand and room-based semi-fixed or permanently mounted physiological patient data acquisition equipment acquiring one or more parameters, monitoring may be performed with small, portable devices (whether as multiple separate sensors or as an integrated acquisition device) coupled to the patient in order to enable the patient to ambulate (e.g., walk) remotely relative to a designated hospital bed or treatment room while maintaining monitoring of the condition of the patient (e.g., heart rhythm, oxygenation, and other patient vital signs). For example, ambulation of the patient may be desirable for resolution of various medical conditions for which the patient is being treated (e.g., chest pain, syncope, post-surgical). A caregiver (e.g., nurse, doctor, or another clinician) may view an output of the monitoring device(s) on the device's user interface, at a remote location such as a patient monitoring central station, via another system such as an Electronic Medical Record (EMR) system, or at a handheld device such as a smart phone or tablet, throughout the duration that the monitoring device(s) is attached or coupled to the patient.

Physiological patient data outputted by the monitoring device(s) may be analyzed by a patient monitoring system to determine whether parameters of the physiological patient data are within desired ranges. If one or more parameters exceed threshold values, the patient monitoring system may generate and display a patient status alert (also referred to herein as an alert) on a display device coupled to the patient monitoring system. For example, the display device could be a bedside monitor, or a smart phone running an app associated with the patient monitoring system.

Effective patient monitoring systems are crucial for timely intervention and improved patient outcomes. However, current systems often face challenges in ensuring prompt caregiver notification, and minimizing false alerts. Current patient monitoring systems often struggle with inaccurate alerts due to pre-defined static thresholds and limited learning capabilities. This can lead to both missed events (false negatives) and unnecessary alerts (false positives), reducing caregiver trust and increasing workloads. The alerts may be generated by one or more artificial intelligence (AI) models, based on clinical guidelines. However, because clinical patient data may vary widely between patients, and may be influenced by many factors, generic alerts produced by the AI models may not be applicable to a specific patient scenario.

For example, an AI model of a patient monitoring system may recommend that an alert be generated for patients whose heart rates exceed a threshold heart rate, based on the clinical guidelines. A first patient may have a heart rate that exceeds the threshold heart rate, and an alert may be generated by the patient monitoring system. A caregiver may determine that the elevated heart rate is a symptom of a medical problem, where the alert may be appropriate. However, a second patient may have a heart rate that exceeds the threshold heart rate, and an alert may be generated by the patient monitoring system. The caregiver may determine that the elevated heart rate of the second patient is due to a medication being taken by the second patient, and not a symptom of a medical problem, whereby the alert may be a false alert. A third patient may have a heart rate that exceeds the threshold heart rate, and an alert may be generated by the patient monitoring system. The caregiver may determine that the elevated heart rate of the third patient is due to anxiety of the second patient, and not a symptom of a medical problem, where the alert may be a false alert. Thus, because each patient's medical scenario is different, and because a number of factors may affect a patient's heart rate, a generic alert may not be applicable to the patient. If a large percentage of alerts are generated when the alerts are not applicable, caregivers may pay less attention to the alerts, and an effectiveness of the alerts may be reduced.

The AI model could be refined, or retrained to account for a wider range of factors. However, a common obstacle to model refinement is a difficulty in generating a sufficient amount of high-quality, labeled data that can account for a variety of patient scenarios. Information about the appropriateness or success of an alert is only available after the alert is generated, and there is currently no efficient way to collect, organize, and store such information for improving models.

To address this issue, an approach is proposed that integrates real-time data analysis, adaptive alert thresholds, multimodal caregiver feedback, and machine learning-driven data labeling to enhance the efficacy of patient monitoring systems and improve the accuracy, reliability, and usability of these systems. The approach enhances the effectiveness of AI-powered patient monitoring systems by addressing the challenges of inaccurate data labeling, high false alert rates, and lack of contextual information.

To ensure accurate data labeling and minimize false alerts, caregivers are provided with easy-to-use acknowledgment mechanisms to confirm receipt of notifications, and categorize alerts as true or false positives. The caregiver may also enter in textual feedback regarding the alert. This caregiver-provided information is then integrated with machine learning models to refine data labeling, improve alert accuracy, and adjust alert thresholds based on patient context and historical data. By integrating real-time data analysis, adaptive alert thresholds, multimodal caregiver acknowledgment, and machine learning-driven data labeling, the disclosed approach aims to streamline the patient monitoring process, enhance the effectiveness of these systems in identifying events, inform clinical decisions, and ultimately improve patient safety and care coordination.

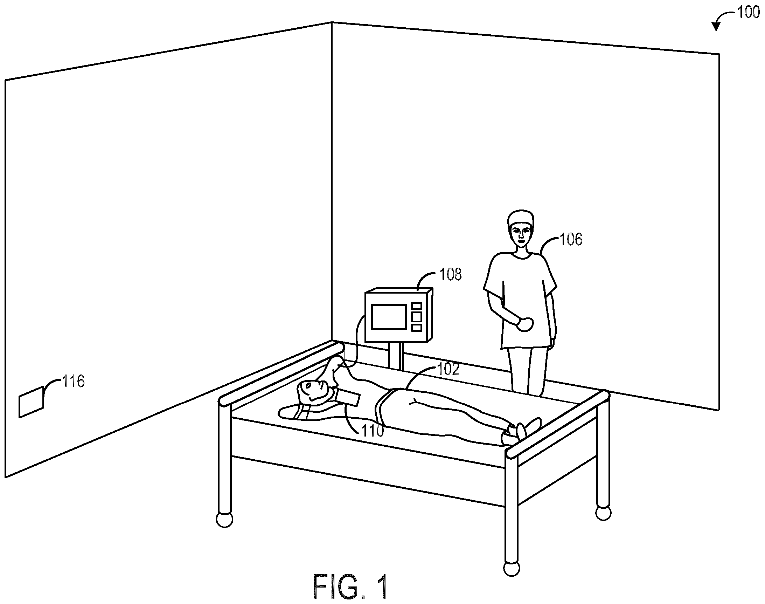

Embodiments of the present disclosure will now be described, by way of example, with reference to the figures. Referring now to FIG. 1, it shows an example patient monitoring environment 100, which in the depicted example is a patient room in a hospital or other medical facility. The patient monitoring environment 100 may include one or more patient monitoring devices or patient data acquisition devices that monitor or acquire one or more physiological patient data, as well as treatment devices. The monitoring environment 100 includes a patient 102 being monitored by a plurality of monitoring devices and also being attended to by a clinician 106. Clinician 106 may be a nurse, physician, medical technologist, or another suitable medical professional. The monitoring devices include a bedside device 108 and a patient monitor 110. In some embodiments, either or both of bedside device 108 and patient monitor 110 may be patient data acquisition devices. The monitoring environment 100 depicts the use of more than one monitoring device but it is understood this is non-limiting as the environment 100 could include one device monitoring one parameter, one device monitoring more than one parameter, multiple devices each monitoring one or more than one parameter, and so on. Due to differing patient conditions and the varying patient monitoring needs, one or more devices may be used to support the monitoring needs of the patient and capable of supporting monitoring in various conditions (e.g., in room, in transport, patient ambulation). The one or more bedside devices 108 may be included or mounted in a floor, table top, or roll stand module that includes one or more leads or other components coupled to the patient or in wireless communication to a device connected to the patient, in order to monitor one or more parameters of the patient (such as ECG, respiration, blood pressure, carbon dioxide levels, etc.). The one or more bedside devices 108 are configured to remain in the patient room, and thus patient 102 may have limited mobility when coupled to the one or more bedside devices 108.

Patient monitor 110 may include one or more telemetry devices (with different sensor capabilities) housed in a common module (as shown) or housed in two or more separate modules. Patient monitor 110 may be positioned on the patient (e.g., via a pouch, holder, or similar, attached to a belt of the patient) or on a movable module (e.g., a wheeled module), such that patient monitor 110 may leave the patient room if the patient leaves the patient room, and may travel with the patient. Patient monitor 110 may be connected to the patient 102 via one or more leads or other components or in wireless communication with an associated sensor, in order to monitor one or more parameters of the patient (such as ECG, respiration, blood oxygen level, etc.).

The patient monitoring data collected by patient monitor 110 may be sent wirelessly (e.g., WiFi, Bluetooth, MBAN) and/or via a hard-wired networked connection to one or more associated devices for processing, analysis, storage, display, etc., such as a central station, patient monitoring database, and/or different patient monitoring system. The methods of wireless communication used by patient monitor 110 and the receiving systems vary widely based on the technology used. In one example communication approach, to facilitate the transfer of the patient monitoring data collected by patient monitor 110, patient monitoring environment 100 and nearby areas (e.g., hallways, closets, open spaces) may include one or more access points 116. Access point 116 may receive and send information (e.g., wirelessly) to patient monitor 110 (e.g., the patient monitoring data, communication status). The access point 116 sends the received information to a processing server, a central station, a telemetry monitoring system, and/or another suitable device. If patient 102 leaves the patient room and moves throughout the medical facility, patient monitoring data collected by patient monitor 110 may be sent to other access points located throughout the medical facility. Patient monitoring data collected by the one or more bedside devices 108 may likewise be sent to a processing and analysis server, the central station, the telemetry monitoring system, and/or another suitable device, via wireless communication with access point 116 or another access point, or via a wired connection. It is understood, this example using a transceiver and access point for data communications is one of many different technologies suitable for sharing acquired patient monitoring data with the associated data processing, analysis, storage, and information viewing system components and infrastructure.

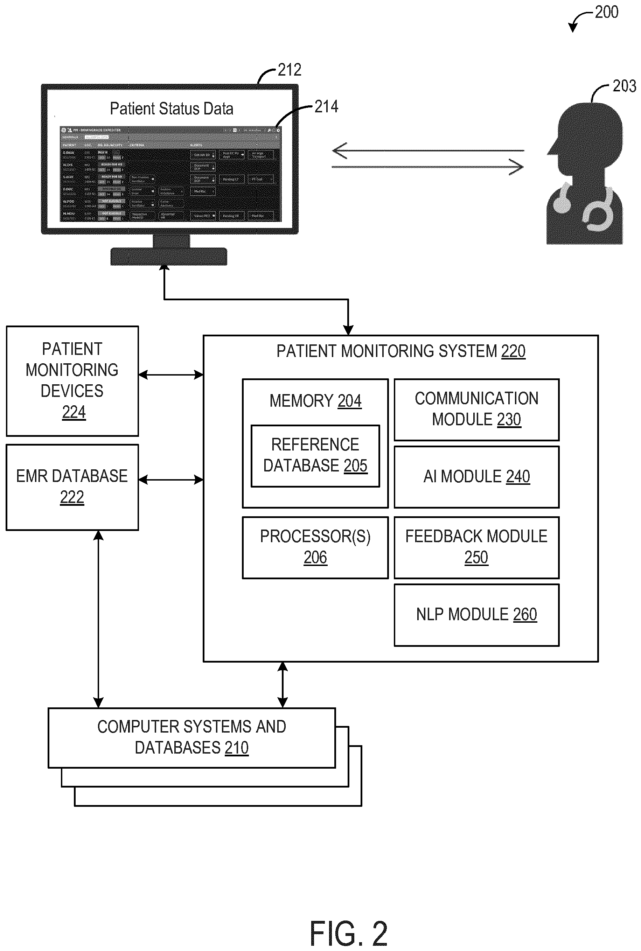

FIG. 2 shows a clinical information system 200 according to an aspect of the disclosure. The clinical information system 200 may include a patient monitoring system 220 configured for generating patient status alerts that may be displayed to a user 203 via a display 212. The display 212 may be any known device having a screen to display the patient status alerts and associated clinical information. The display device 212, and in some examples, more than one display device, may be communicatively coupled to patient monitoring system 220.

The display device 212 may include a processor, memory, communication module, user input device, display (e.g., screen or monitor), and/or other subsystems and may be in the form of a desktop computing device, a laptop computing device, a tablet, a smart phone, or other device. The display device 212 may be adapted to send and receive encrypted data and display medical information, including medical images in a suitable format such as digital imaging and communications in medicine (DICOM) or other standards. The display device 212 may be located locally at a medical facility (such as in a care unit of the medical facility) and/or remotely from the medical facility (such as a caregiver's mobile device). In some embodiments, the display device 212 may be a non-limiting example of patient monitor 110 and/or bedside device 108.

The patient status alerts may be viewed via a user interface (UI) 214 (which may be a graphical user interface and thus also referred to as a GUI) of the display device 212. The patient status alerts may comprise visual or graphical elements and/or sounds (e.g., an alert). When viewing the patient status alerts via the UI 214, the user may enter input (e.g., via a user input device, which may include a keyboard, mouse, microphone, touch screen, stylus, or other device) that may be processed by the patient monitoring system 220. In examples where the user input is a selection of a link or control button of the UI 214, the user input may trigger display of additional clinical information relevant to a patient status, or other actions.

The clinical information system 200 may include an EMR database 222, which may be communicatively coupled to the patient monitoring system 220. EMR database 222 may be stored in a mass storage device configured to communicate with secure channels (e.g., HTTPS and TLS), and store data in encrypted form. Further, the EMR database/computing system may be configured to control access to patient electronic medical records such that only authorized caregivers may edit and access the electronic health records. An EMR for a patient may include medical device data, including historical patient monitoring data (e.g., vital signs measured or otherwise ascertained by medical devices such as heart rate, oxygen saturation, etc.), user-specified medical parameters (e.g., acuity scores, pain scores), patient demographic information, family medical history, past medical history, lifestyle information, preexisting medical conditions, current medications, allergies, surgical history, past medical screenings and procedures, past hospitalizations and visits, etc.

In addition to the EMR database 222, historical patient information that is integrated into the patient status alerts may also be stored in one or more computer systems and databases 210 in communication with patient monitoring system 220 and/or the EMR database 222. For example, the computer systems and databases 210 may include a picture archiving and communication system (PACS) that may store and communicate medical images and associated reports (e.g., clinician findings), such as ultrasound images, MRI images, etc., according to the DICOM format. The computer systems and databases 210 may include a radiology information system (RIS), which may store radiology images and associated reports, such as CT images, X-ray images, etc. The computer systems and databases 210 may include a laboratory database and/or a pathology database, which may store lab results, pathology images and related reports, including visible light or fluorescence images of tissue, such as immunohistochemistry (IHC) images. It should be appreciated that the example computer systems and databases provided herein are for illustrative purposes, and in various embodiments, additional, fewer, or different computer systems and/or databases may be included without departing from the scope of this disclosure.

Patient monitoring system 220 may include a memory 204 and a processor(s) 206 to store and execute the methods disclosed herein to generate the patient status alerts and patient status data, as well as send and receive communications, graphical user interfaces, medical data, and other information. Memory 204 may include one or more data storage structures, such as optical memory devices, magnetic memory devices, or solid-state memory devices, for storing programs and routines executed by processor(s) 206 to carry out various functionalities disclosed herein. Memory 204 may include any desired type of volatile and/or non-volatile memory such as, for example, static random access memory (SRAM), dynamic random access memory (DRAM), flash memory, read-only memory (ROM), etc. Processor(s) 206 may be any suitable processor, processing unit, or microprocessor, for example. Processor(s) 206 may be a multiprocessor system, and, thus, may include one or more additional processors that are identical or similar to each other and that are communicatively coupled via an interconnection bus.

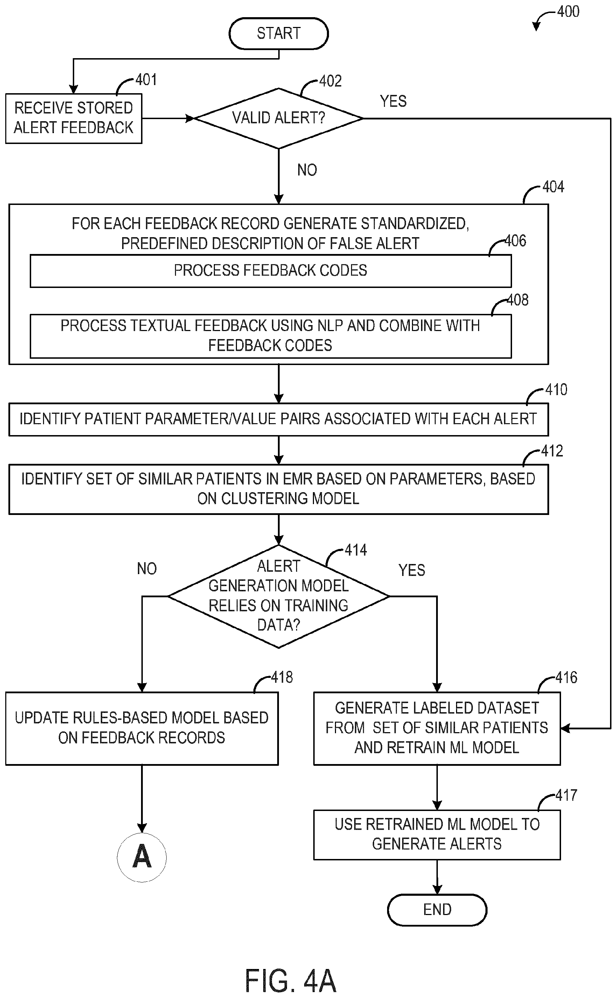

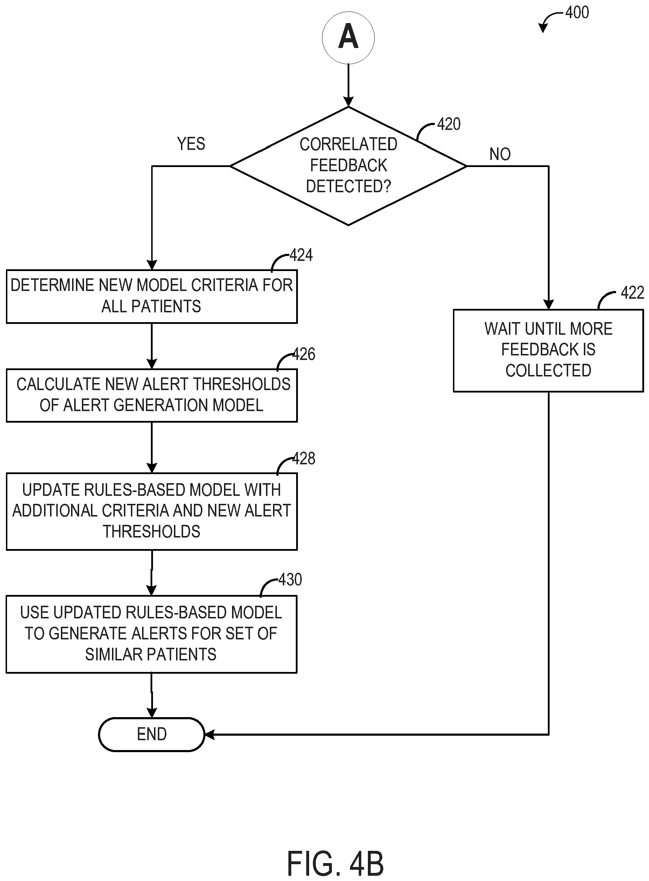

In particular, memory 204 may include a reference database 205, which may store a list of standardized, predefined descriptions that may be used to describe a false alert generated by an AI model of patient monitoring system 220. The standardized, predefined descriptions may be used to determine a correlation between false alerts generated for different patients, for refining the AI model, as described below in reference to FIGS. 4A and 4B.

In some examples, patient monitoring system 220 may be implemented over a cloud or other computer network. For example, patient monitoring system 220 is shown in FIG. 2 as constituting a single entity, but it should be appreciated that patient monitoring system 220 may be distributed across multiple devices, such as across multiple servers.

Patient monitoring system 220 may be configured to generate and output one or more patient status alerts for display via patient monitoring devices 224, display 212, and/or a different wired or wireless computing device, such as a smart phone of a caregiver. Each patient status alert may include real-time patient monitoring data including clinical markers (also referred to as criteria) relevant to the patient status alert, as well as historical physiological patient data related to the real-time patient monitoring data. The historical physiological patient data may be automatically extracted from the EMRs and/or other medical information of the patient (e.g., pathology reports, imaging scans/reports, etc.) that may or may not be stored in the EMRs when the patient status alert is first launched and/or configured.

As used herein, the term "module" may include a hardware and/or software system that operates to perform one or more functions. For example, a module may include a computer processor, controller, or other logic-based device that performs operations based on instructions stored on a tangible and non-transitory computer readable storage medium, such as a computer memory. Alternatively, a module may include a hard-wired device that performs operations based on hard-wired logic of the device. Various modules shown in the attached figures may represent the hardware that operates based on software or hardwired instructions, the software that directs hardware to perform the operations, or a combination thereof.

"Modules" may include or represent hardware and associated instructions (e.g., software stored on a tangible and non-transitory computer readable storage medium, such as a computer hard drive, ROM, RAM, or the like) that perform one or more operations described herein. The hardware may include electronic circuits that include and/or are connected to one or more logic-based devices, such as microprocessors, processors, controllers, or the like. These devices may be off-the-shelf devices that are appropriately programmed or instructed to perform operations described herein from the instructions described above. Additionally or alternatively, one or more of these devices may be hard-wired with logic circuits to perform these operations.

Patient monitoring system 220 may include a communication module 230. Communication module 230 may facilitate transmission of electronic data within and/or among one or more systems. Communication via communication module 230 can be implemented using one or more protocols. In some examples, communication via communication module 230 occurs according to one or more standards (e.g., Digital Imaging and Communications in Medicine (DICOM), Health Level Seven (HL7), ANSI X12N, etc.). Communication module 230 can be a wired interface (e.g., a data bus, a Universal Serial Bus (USB) connection, etc.) and/or a wireless interface (e.g., radio frequency, infrared, near field communication (NFC), etc.). For example, communication module 230 may communicate via wired local area network (LAN), wireless LAN, wide area network (WAN), etc. using any past, present, or future communication protocol (e.g., BLUETOOTH^{™}, USB 2.0, USB 3.0, etc.).

Patient monitoring system 220 may include a AI module 240. The AI module 240 may store one or more models that may be used to generate the patient status alerts. The one or more models may include a rules-based system, where machine-implementable rules may be applied to a set of patient data relating to a current or prior status of the patient retrieved by the patient monitoring system 220. The one or more models may also include machine learning (ML) or deep learning (DL) models trained to output an indicator of patient status, based on various input data.

Patient monitoring system 220 may include a feedback module 250. The feedback module 250 may be used to request and receive feedback from a caregiver with respect to alerts generated by patient monitoring system 220. Patient monitoring system 220 may also include a natural language processing (NLP) module 260. The NLP module 260 may be used to extract insights from caregiver explanations provided as feedback to the alerts.

While not specifically shown in FIG. 2, additional devices described herein (e.g., display device 212) may likewise include user input devices, memory, processors, and communication modules/interfaces similar to communication module 230, memory 204, and processor(s) 206 described above, and thus the description of communication module 230, memory 204, and processor(s) 206 likewise applies to the other devices described herein. As an example, the display device 212 may store user interface templates in memory that include placeholders for relevant information stored on patient monitoring system 220 or received via patient monitoring system 220, which a user of display device 212 may configure. The patient status alerts and relevant patient information may be retrieved from patient monitoring system 220 and inserted in the placeholders. The user input devices may include keyboards, mice, touch screens, microphones, or other suitable devices.

In various examples, the caregiver may open the patient monitoring system by first logging into a patient management system of a healthcare network, and selecting a patient monitoring system icon, tile, or similar menu listing of one or more options for retrieving patient data of the patient management system to start the patient monitoring system. In other examples, the patient monitoring system may be launched from within a different computer system of a hospital, such as an EMR system, where the patient monitoring system may be listed as a selectable menu option within a UI of the EMR system. In general, the patient monitoring system may be integrated into any suitable existing care device of a healthcare or hospital system.

In some embodiments, patient monitoring system 220 may be launched by a caregiver when the caregiver wishes to assess the status of one or more patients. In other embodiments, the patient monitoring system may be continuously running, and elements of the patient monitoring system and/or patient data may be periodically updated automatically by the patient monitoring system. For example, the caregiver may start the patient monitoring system and configure a patient status alert to be generated for a patient.

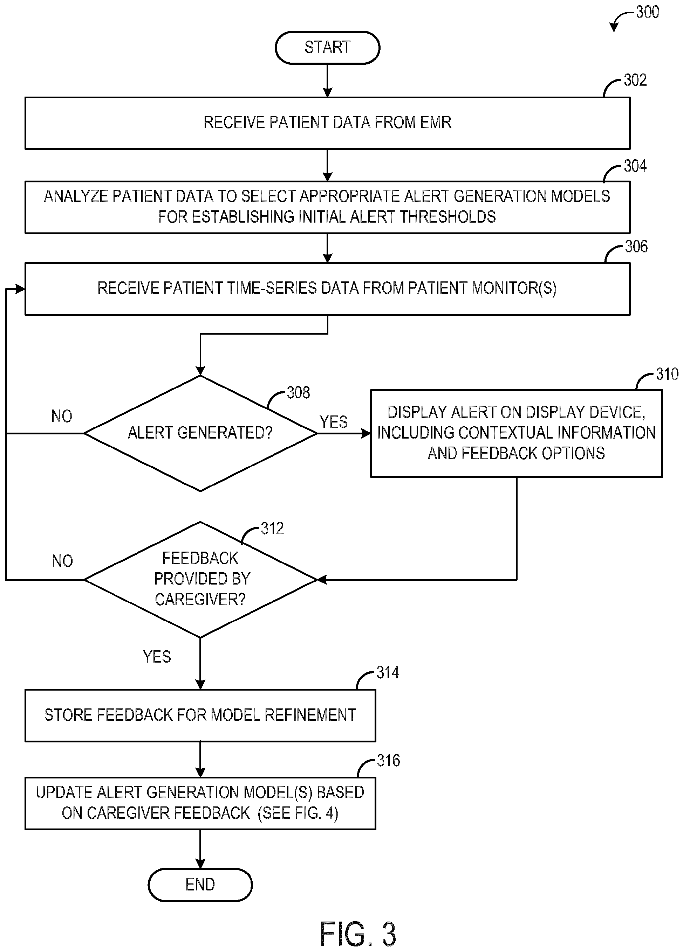

Referring now to FIG. 3, a method 300 is shown that illustrates how the patient monitoring system 220 may be used to generate alerts and receive feedback from caregivers for AI model refinement is shown. Method 300 may be carried out by a patient monitoring system, such as the patient monitoring system 220 of FIG. 2. Method 300 and other methods described herein may be performed according to instructions stored in a memory (e.g., memory 204) of the patient monitoring system, which may be executed by a processor (e.g., processor(s) 206) of the patient monitoring system.

Method 300 begins at 302, where method 300 includes receiving patient data from one or more electronic medical records (EMR). The patient data may be generated for a single patient, or a plurality of patients of a care unit of a healthcare organization or network (e.g., a hospital), such as an ICU. While method 300 is described in reference to a single patient, it should be appreciated that the method may be applied to a plurality of patients in a concurrent or sequential manner, in some embodiments. In various embodiments, the patient data may be retrieved from the EMR in accordance with instructions by a caregiver or other user.

To retrieve the patient data, the user may open a patient monitoring system application running on a computer or network terminal of the ICU or on the patient monitor and select one or more suitable controls of a UI of the patient monitoring system. For example, a clinician may select a button in a UI displayed on a display device (e.g., display device 212 of FIG. 2) to request a patient status alert be set up in accordance with the patient data. In some embodiments, the user may initiate an alert as part of a routine procedure, (e.g., a daily procedure) in accordance with one or more clinical guidelines of the healthcare organization, as part of routine patient management practices. In other embodiments, the user may initiate an alert in response to a desire to assess the status of a specific patient.

At 304, method 300 includes analyzing the patient data to select one or more suitable alert generation models for establishing initial alert thresholds for the patient. The alert generation models may be selected by an AI model (e.g., a rules-based model) based on the analysis of the patient data. For example, the rules-based model may take elements of the patient data as inputs, and may output a first appropriate alert generation model for generating a first alert, based on a first combination of patient parameters; a second appropriate alert generation model for generating a second alert, based on a second combination of patient parameters; a third appropriate alert generation model for generating a third alert, based on a third combination of patient parameters; and so on. The alert generation models may include various types of models, including statistical or probabilistic models (e.g., decision trees, Bayesian networks, etc.), AI models such as rules-based models or expert systems, ML models such as convolutional neural networks (CNNs), recurrent neural networks (RNNs), or other types of neural networks, or other types of models. Each of the alert generation models may establish thresholds or acceptable ranges of the patient parameters, where if a threshold is exceeded, an alert may be generated.

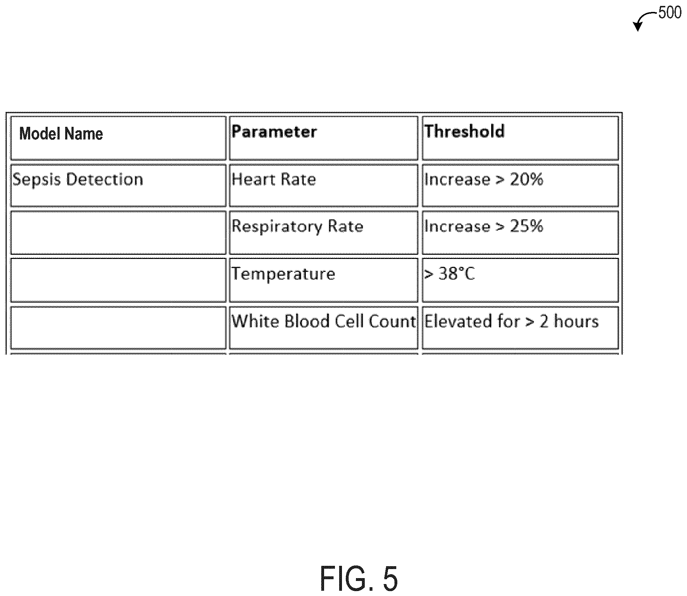

Referring briefly to FIG. 5, an exemplary alert generation model 500 is shown, in accordance with an embodiment. Alert generation model 500 is encoded as a list of parameters and corresponding thresholds for the parameters, where if the thresholds are exceeded, alert generation model 500 may generate an alert. Alert generation model 500 is a sepsis detection model that assesses a possibility of a patient experiencing sepsis, based on four parameters: heart rate, respiratory rate, temperature, and white blood cell count. Each parameter of the four parameters may be received by the patient monitoring system as time-series data from one or more patient monitors coupled to the patient. In accordance with alert generation model 500, an alert may be generated if the heart rate of the patient increases above 20% of a resting heart rate, the respiratory rate increases above 25% of a resting respiratory rate, the temperature of the patient increases above 38°C, and the white blood cell count is above a threshold amount for longer than two hours.

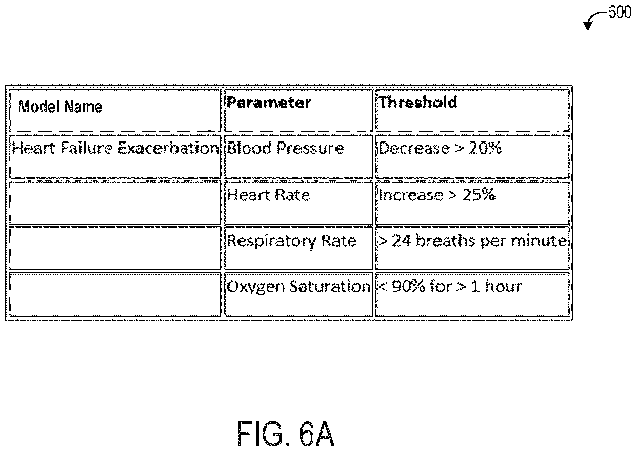

FIG. 6A shows a second, similar exemplary alert generation model 600, where alert generation model 600 is encoded as a list of parameters and corresponding thresholds for the parameters, where if the thresholds are exceeded, alert generation model 650 may generate an alert. Alert generation model 600 is a heart failure exacerbation model that assesses a degree of heart failure exacerbation, based on four parameters: blood pressure (BP), heart rate (HR), respiratory rate (RR), and oxygen saturation (SpO2). As with model 500, each parameter of the four parameters may be received by the patient monitoring system as time-series data from one or more patient monitors coupled to the patient. In accordance with alert generation model 600, an alert may be generated if the blood pressure of the patient decreases by 20%, the heart rate of the patient increases above 25% of a resting heart rate, the respiratory rate increases above 24 breaths per minute, and the SPO2 of the patient decreases below 90% for more than one hour. It should be appreciated that alert generation models 600 and 650 are just two of many types of models; in other embodiments, an alternative alert generation model may be a machine learning (ML) model, such as a neural network, a decision tree model, a Bayesian network, etc.

Returning to method 300, at 306, method 300 includes receiving time-series patient data from one or more patient monitors (e.g., patient monitoring devices 224) coupled to the patient monitoring system. The alert generation models may each take certain types of time-series patient data acquired by a patient monitor of the one or more patient monitors as input, and may output an indication of whether an alert should be generated based on the time-series patient data. For example, in the case of the heart failure exacerbation model shown in FIG. 6A, the time-series patient data may include a BP of the patient, an HR of the patient, an RR of the patient, and an SpO2 of the patient. Each alert generation model may specify a set of patient parameters of the time-series patient data that are used by the alert generation model, and the patient monitoring system may request or retrieve the relevant sets of patient parameters from the time-series patient data outputted by the one or more patient monitors based on the selection of alert generation models.

If measured values of parameters of the time-series patient data (e.g., physiological patient data) deviate from an expected range of the parameter values, the alert generation model may output an indication that an alert should be generated and displayed to a caregiver. For example, if the BP, HR, RR, and SpO2 of the patient increase or decrease below respective threshold amounts, a heart failure exacerbation model that monitors the patient may generate an alert.

At 308, method 300 includes determining whether an alert has been generated, based on an output of the one or more alert generation models. If an alert has not been generated, method 300 proceeds back to 306, to continue to receive the patient time-series data. Alternatively, if an alert has been generated, method 300 proceeds to 310.

At 310, the method includes displaying the alert on the display device. The alert may include contextual information related to the alert, and may be accompanied by beep, tone, or similar audio output. The characteristics and features of the alert may vary across different implementations.

In particular, the displayed alert may include one or more controls to display a feedback collection panel, or may automatically display the feedback control panel, which may be used by a caregiver to enter in feedback with respect to the generation of the alert. Using the feedback panel, the caregiver may enter in various types of feedback about the applicability, appropriateness, correctness, and/or accuracy of the alert. After the feedback has been provided, for example, via a "save" button, the feedback may be stored in the patient monitoring system, and the feedback collection panel may be closed.

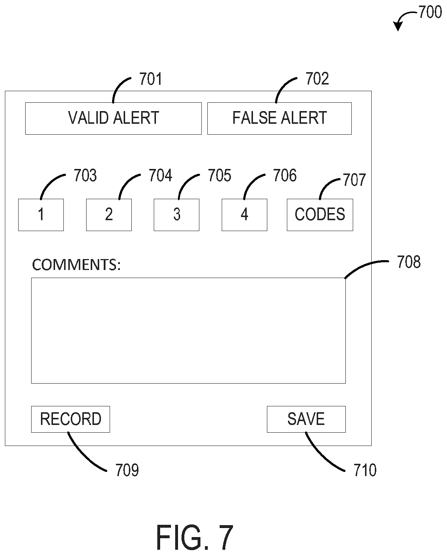

Turning briefly to FIG. 7, an exemplary feedback collection panel 700 of a patient monitoring system is shown, which may be automatically displayed when an alert is generated or when requested by a caregiver. Feedback collection panel 700 may be displayed by selecting a control element such as a virtual button displayed on a display screen of a patient monitor, a physical button on the patient monitor, a button on a screen of a computing device, such as a smart phone app, via a voice command, or in another manner. In some embodiments, the alert may be terminated/silenced when the caregiver displays feedback collection panel 700. By linking the termination of the alert with the display of feedback collection panel 700, the caregiver may be encouraged to provide feedback regarding the alert to the patient monitoring system.

Feedback collection panel 700 may include a graphical user interface (GUI) with various graphical elements which may be selected by a user to provide feedback on the alert to the patient monitoring system. In the depicted embodiment, feedback collection panel 700 includes a valid alert button 701, and a false alert button 702, which may be used by the caregiver to acknowledge and/or confirm receiving the alert. By selecting the valid alert button 701, the caregiver can indicate to the patient monitoring system that the alert was valid, meaning, the alert correctly indicated a medical concern regarding the patient worthy of notifying the caregiver. By selecting the false alert button, the caregiver can indicate to the patient monitoring system that the alert was a false alert, meaning that the alert did not indicate a medical concern regarding the patient worthy of notifying the caregiver. Thus, at a simplest level, feedback collection panel 700 can collect true positive/false positive data with respect to alerts via a single selection action. The true positive/false positive data may be used to refine one or more alert generation models relied on by the patient monitoring system to generate the alerts, as described below.

Feedback collection panel 700 may also include a plurality of numbered code buttons, such as buttons 703, 704, 705, and 706, which allow the caregiver to provide predefined feedback messages regarding the alert in accordance with a respective plurality of stored codes. In other words, by pressing button 703, a predefined feedback message associated with a selected code (e.g., "1") may be submitted to the patient monitoring system; by pressing button 704, a predefined feedback message associated with a selected code (e.g., "2") may be submitted to the patient monitoring system; and so on. The codes may be defined in advance by caregivers, hospital administrators, managers of the patient monitoring system, or a combination of the three and/or other organizations or individuals. A code reference button 707 may be included in feedback collection panel 700, which when selected may display a pop-up panel with a reference list of the codes, for example.

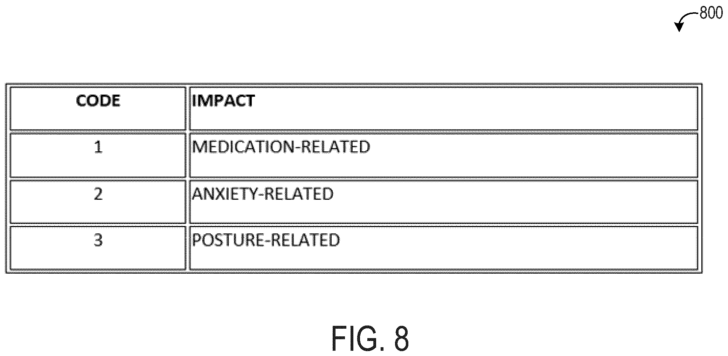

Referring briefly to FIG. 8, a code reference list 800 is shown of an exemplary set of codes that may be included in feedback provided by the caregiver via feedback collection panel 700, which may be displayed on the pop-up panel for quick reference. Code reference list 800 includes three codes. A first code "1" indicates that a false alert may be attributable to issues related to medication; a second code "2" indicates that the false alert may be attributable to issues related to patient anxiety; and a third code "3" indicates that the false alert may be attributable to issues related to a posture or position of the patient. For example, if an alert is generated because a patient's HR is higher than a threshold HR, and the caregiver records the alert as a false alert because a medication the patient was taking caused the HR to increase, the caregiver may include the code "1". If the caregiver records the alert as a false alert because the patient was anxious, causing the HR to increase, the caregiver may include the code "2". If the caregiver records the alert as a false alert because the patient was in a position that caused the patient to exert themselves, causing the HR to increase, the caregiver may include the code "3". It should be appreciated that the examples included in code reference list 800 are for illustration and not limitation, and in other embodiments, code reference list 800 may include different codes that represent different concepts, and/or a greater or lesser number of codes, without departing from the scope of this disclosure.

Returning to FIG. 7, feedback collection panel 700 may include a comments field 708, where the caregiver can enter in textual feedback messages that may be more detailed, comprehensive, and/or customized to the patient than the messages associated with the codes. In some cases, a textual feedback message may include new proposed threshold values for one or more parameters used by the alert generation model, and/or new proposed parameters for consideration by the alert generation model. For example, if feedback provided regarding an alert generated by the heart failure exacerbation model 600 of FIG. 6A indicates that the model incorrectly generated an alert because the model did not take into consideration that the patient was a diabetic, the textual feedback may include a recommendation to include an additional criterion for diabetes in the heart failure exacerbation model 600, as described in greater detail below.

Additionally or alternatively, the caregiver may include voice feedback messages via a record button 709, which may activate a microphone that allows the voice feedback messages to be stored in the memory of the patient monitoring system. When the caregiver is finished providing feedback, the caregiver may save the feedback to the patient monitoring system and close feedback collection panel 700 by selecting a save button 710.

In some embodiments, feedback collection panel 700 may be customized and/or displayed at different times and/or in different ways, to collect specific information from users. For example, input options for soliciting/providing feedback may be embedded into alert notifications, patient treatment dashboards, and/or other computer applications running in a hospital or health care environment. The patient monitoring system could provide real-time data visualization tools and actionable insights to caregivers within feedback collection panel 700. This could include trend charts, predictive models, and personalized recommendations, enabling caregivers to quickly grasp the patient's condition and make informed decisions. Further, the patient monitoring system can proactively prompt caregivers for input at specific times, or when events occur, to ensure that important information is captured promptly. For example, when an alert is generated, a pop-up display panel may prompt the user to enter in feedback regarding the alert.

In some embodiments, historical patterns of one or more patients' parameters may be analyzed to determine when feedback collection panel 700 might be displayed to caregivers. For example, if historical patient data indicates a history of a variance in a blood pressure of the patient greater than a threshold variance, when an alert is displayed as a result of the patient's blood pressure exceeding a threshold blood pressure, the patient monitoring system may display feedback collection panel 700 and prompt the user to indicate whether the alert is accurate.

It should be appreciated that feedback collection panel 700 is shown for illustration and not limitation. In other embodiments, feedback may be collected from users in other ways without departing from the scope of this disclosure. For example, in some embodiments, touchscreen gestures (e.g., tapping, swiping, etc.) may be used to provide input quickly and efficiently. In other embodiments, adaptive input forms may be used, which may be automatically generated based on measured patient parameter values, and which may present relevant questions and prompts based on specific needs of the patient.

Returning to FIG. 3, at 312, method 300 includes determining whether feedback has been provided by a caregiver, via the feedback options of the feedback control panel. If feedback is not provided, method 300 proceeds back to 306, to continue to receive the patient time-series data. If feedback has been provided by the caregiver, method 300 proceeds to 314.

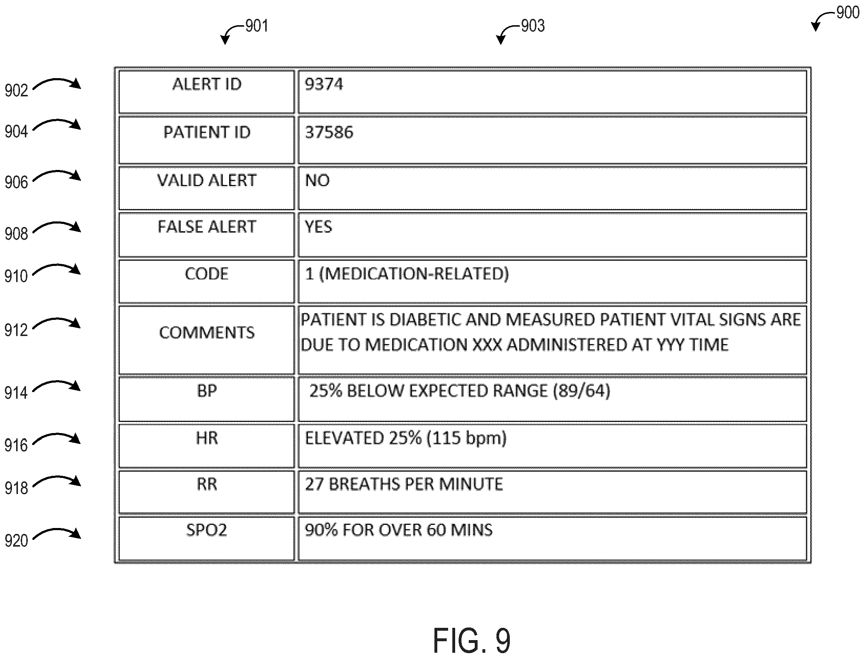

At 314, method 300 includes storing the feedback in a memory (e.g., memory 204) of the patient monitoring system. The stored feedback may be encoded as a feedback record in the memory of the patient monitoring system, as described below in reference to FIG. 9. After the caregiver feedback is stored, at 316, method 300 includes updating the one or more alert generation models based on the caregiver feedback, as described below in reference to FIGS. 4A and 4B.

FIG. 9 shows an exemplary feedback record 900 that may be stored in the patient monitoring system, as a result of the caregiver submitting feedback on an alert generated by the patient monitoring system via feedback collection panel 700 of FIG. 7. In the depicted embodiment, feedback record 900 is stored as a table with two columns. A first column 901 includes data labels, and a second column 903 includes feedback data and/or alert data associated with the labels.

Feedback record 900 includes ten rows of feedback data. A first row 902 shows an alert identifier, which may be used to identify the alert that was generated. More specifically, the alert identifier may be used to determine which alert generation model generated the alert. In this way, the feedback information included in feedback record 900 may be used to refine or increase an accuracy of the associated alert generation model. A second row 904 shows a patient identifier, which may be used to identify the patient. A third row 906 and a fourth row 908 indicate whether the caregiver describes the alert as a valid alert or a false alert, respectively. For example, fourth row 908 indicates that the caregiver provided feedback that the alert was a false alert.

Feedback record 900 may include one or more codes selected by the caregiver, and the feedback messages associated with the codes. For example, a fifth row 910 of feedback record 900 indicates that the caregiver entered in the code "1", which indicates, in this example, that the cause of the false alert was a medication that the patient was on. A comment clarifying the code is included a sixth, textual feedback row 912 of feedback record 900, where the caregiver specifies that the patient's measured vital signs were outside of expected ranges as a result of a medication administered to the patient for diabetes, as opposed to a medically concerning scenario. In the event that the caregiver records a voice feedback message, the voice feedback message may be automatically transcribed as a textual feedback message and stored in row 912.

Feedback record 900 may include contextual information regarding the alert, such as measurements of the patient time-series data that may have been a cause of generating the false alert indicated at row 908. For example, if the alert is generated by the heart failure exacerbation model 600 of FIG. 6A, the alert may have been generated as a result of a blood pressure (BP) of the patient being more than 20% below an expected range; a heart rate (HR) of the patient exceeding a resting heart rate of the patient by 25%, a respiratory rate (RR) of the patient exceeding a resting respiratory rate of the patient by more than 24 breaths per minute, and a measured SpO2 being below 90% for over an hour. When the caregiver provides feedback on the alert, the measurements of the blood pressure, heart rate, respiratory rate, and SpO2 of the patient at the time the alert is generated may be included in feedback record 900, to validate the operation of the heart failure exacerbation model. Specifically, a seventh row 914, an eighth row 916, a ninth row 917, and a tenth row 920 may indicate measured values of BP, HR, RR, and SpO2, respectively, and/or indications of a degree of change in the values at the time that the alert was generated.

Referring now to FIG. 4A, a method 400 is shown for refining or updating an alert generation model based on feedback regarding alerts generated by the alert generation model submitted by a caregiver in response to the alerts, according to an embodiment of the disclosure. Method 400 may be carried out by the patient monitoring system described in method 300 of FIG. 3 and FIG. 2. In various embodiments, method 400 may be performed periodically, for example, when a threshold amount of feedback has been collected, where the threshold amount is a sufficient amount for retraining the alert generation model. It should be appreciated that in different embodiments, one or more steps of method 400 may be omitted or performed in a different order, without departing from the scope of this disclosure. Additionally, in some embodiments, a first portion of method 400 may be performed at a first time, and a second portion of method 400 may be performed at a second, later time.

Method 400 begins at 401, where method 400 includes receiving stored alert feedback records submitted by a plurality of caregivers. The stored alert feedback record may include data described in reference to FIG. 9 above, for example.

At 402, method 400 includes determining, for each feedback record of the received stored alert feedback records, whether the feedback record indicates that a respective alert was a valid alert. If at 402 it is determined that the alert was a valid alert, method 400 proceeds to 416, where the valid alert data may be used in the generation of a labeled dataset for retraining the alert generation model, as described below. Alternatively, if at 402 it is determined that the alert was not a valid alert, then it may be inferred that the alert was a false alert, whereby method 400 proceeds to 404.

At 404, method 400 includes, for each received feedback record including a false alert, generating a standardized, predefined description of the false alert that may be used to combine and compare the received feedback records. The standardized, predefined description of the false alert may be generated by a feedback module of the patient monitoring system, such as feedback module 250 of FIG. 2.

At 406, generating the standardized, predefined description of the false alert includes processing one or more feedback codes included in the feedback record. The feedback codes may be submitted by caregivers via buttons on a feedback collection panel generated by the patient monitoring system, as described above in reference to method 300. Processing the feedback codes may include retrieving the feedback messages associated with the codes included in the feedback record.

At 408, generating the standardized, predefined description of the false alert includes processing textual feedback provided by the caregiver in the feedback records. In some embodiments, the textual feedback may be processed by an NLP module of the patient monitoring system, such as NLP module 260. In some embodiments, the textual feedback may be generated from audio recordings made by the caregivers. That is, the caregivers may optionally include audio feedback via a microphone of the patient monitoring system, and the audio feedback may be automatically transcribed into the textual feedback for processing by the NLP module.

During processing of the textual feedback, keywords or phrases included in the textual feedback may be identified using one or more lookup tables, and extracted from the textual feedback. The extracted keywords or phrases and the feedback messages associated with feedback codes used may then be combined and mapped to a corresponding standardized, predefined description stored in a reference database (e.g., reference database 205) of the patient monitoring system. The predefined description may define a category that the false alert may be classified to. For example, the false alert of feedback record 900 may be categorized as "False alert due to medication of class XXX administered to patient for condition YYY". The standardized, predefined description may then be added to and stored with the feedback record.

In other words, method 400 includes extracting information, including both structured data (e.g., the coded feedback messages) and unstructured data (e.g., the extracted keywords and phrases) from the feedback record, and then converting that information into a standard format. The standard format may be used to compare and/or group patients associated with different feedback records into meaningful groups (e.g., groups that share a same standardized, predefined description of a false alert) that may be used to refine the alert generation model, as described in greater detail below.

At 410, method 400 includes extracting a set of patient data parameter/value pairs associated with each feedback record received. For example, for the heart failure exacerbation model described in reference to FIG. 6A, the patient parameters include the blood pressure, the heart rate, the respiratory rate, and the SpO2 of the patient. For each of the patient parameters, values (e.g., measurements) may be recorded in the feedback records that correspond to the time the alert was generated.

At 412, method 400 includes identifying a set of similar patients in an EMR database coupled to the patient monitoring system, where the similar patients are similar to the patients for whom the alerts that prompted the feedback were generated. The similar patients may be defined as patients who have data parameters and/or characteristics that are the same as or within a same range as the patients for whom the alerts that prompted the feedback were generated. In the example of the heart failure exacerbation model, the similar patients may be patients having similar BP, HR, RR, and SpO2 ranges, who are diabetic, or who are on a similar medication as the patient of the feedback record. In various embodiments, the set of similar patients may be classified using a clustering model, or a different statistical method.

In other words, a first set of patients may be identified and selected from feedback records indicating a false alert, where the first set of patients share a same standardized, predefined description of the false alert; and a second set of patients may be selected from a general population of patients of the EMR, where the second set of patients share one or more similar or defining characteristics with the first set of patients (e.g., as determined by a statistical technique such as a clustering model). The second set of patients may include the first set of patients. Because the second set of patients has characteristics and patient data that are representative of the first set of patients, data from the second (e.g., larger) set of patients may be used to refine the alert generation model, as described below, where data of the first set of patients may not be sufficient to refine the alert generation model.

At 414, method 400 includes determining whether the alert generation model relies on training pair data for training, where an accuracy of the ML model may be increased by further training on a training dataset. If the alert generation model relies on training pair data, method 400 proceeds to 416.

At 416, method 400 includes generating a labeled, training dataset to refine the alert generation model. The labeled training dataset may include a first plurality of training pairs, where each training pair of the first plurality of training pairs may include patient data of a different patient. For example, a training pair of the first plurality of training pairs may include vital sign measurements of a patient extracted from a feedback record generated for an alert that was described as valid by a caregiver (e.g., received from step 402) as input data, and an encoding of the validity of the alert (from the feedback record) as ground truth data. For example, a "0" may be used to represent a valid alert. The first plurality of training pairs may also include vital sign measurements of patients extracted from an EMR database (e.g., EMR database 222) that fall within typical ranges of vital sign measurements (e.g., BP, HR, RR, SpO2, etc.) as input data, and the encoding for the valid alert as ground truth data.

Generating the labeled training dataset may include generating a second plurality of training pairs, where the second plurality of training pairs includes patient data of the second, similar set of patients (also including patient data included in feedback records generated on alerts that were described as false alerts by caregivers). For example, a training pair of the second plurality of training pairs may include vital sign measurements of a patient extracted from a feedback record generated for an alert that was described as a false alert by a caregiver as input data, and an encoding of the false alert as ground truth data. For example, a "1" may be used to represent a false alert. The second plurality of training pairs may also include vital sign measurements of patients of the similar set of patients extracted from an EMR database as input data, and the encoding for the false alert as ground truth data.

Additionally, the input data of training pairs of both of the first and second plurality of training pairs may include an encoding of data provided in one or more codes and/or textual feedback provided in a respective feedback record. For example, if the patient is a diabetic, an encoding for diabetes may be included in the input data of the second plurality of training pairs, and a corresponding encoding for not having diabetes may be included in the input data of the first plurality of training pairs. For example, a boolean (e.g., 0 or 1) may be used, as with the ground truth data.

The first plurality of training pairs and the second plurality of training pairs may then be combined in a random order, and the alert generation model may be trained on the resulting training dataset using supervised or semi-supervised learning. Additionally or alternatively, in some embodiments, the alert generation model may be trained using reinforcement learning. By training the alert generation model in this manner, an accuracy of the alert generation model may be increased, in particular, with respect to new criteria added to the alert generation model. At 417, method 400 includes using the retrained ML model to generate alerts, and method 400 ends.

Alternatively, if at 414 the alert generation model does not rely on training data pairs, it may be inferred that the alert generation model is a rules-based model, such as a decision tree (e.g., as in the heart failure exacerbation model of FIG. 6A), whereby method 400 proceeds to 420.

At 418, method 400 includes updating the rules-based model based on the feedback records. Updating the rules-based model based on the feedback records is described below in reference to FIG. 4B, where method 400 continues.

Referring now to FIG. 4B, method 400 continues at 420. At 420, method 400 includes determining whether the received feedback records include correlated feedback, above a threshold level. The correlated feedback may be detected when a threshold number or percentage of feedback records include the same standardized, predefined description assigned at step 408. In other words, the categorical descriptions that the feedback records are classified into may be used to associate or pool feedback from various patients. For example, if 1000 feedback records are collected from alerts generated by the patient monitoring system, and at least 10% of the records include the same standardized, predefined description generated at 404, then the 10% of the feedback records would be considered correlated feedback.

If at 420 no significant correlations are detected between the received feedback records, then it may be inferred that an amount of feedback data collected is not sufficient to update the rules-based model, and method 400 proceeds to 422. At 422, method 400 includes waiting until more feedback is collected. The rules-based alert generation model is not updated, and method 400 ends.

If at 420 the number or percentage of correlated feedback records detected is greater than the threshold number or percentage, it may be inferred that sufficient feedback data has been collected to update the rules-based alert generation model, whereby method 400 proceeds to 424.

At 424, method 400 includes determining new criterion to be included in the alert generation model for all patients, meaning, new conditions to be imposed by the alert generation model for generating an alert. Specifically, based on the standardized, predefined descriptions of the detected correlated feedback records, one or more additional criteria may be included in the model that may increase an accuracy of the model. The one or more additional criteria may be extracted from the standardized, predefined description of the correlated feedback records. The one or more additional criteria of the model may include an additional health parameter measurement (e.g., temperature, etc.); an additional characteristic of the patient (e.g., age, sex, etc.); a class of medication the patient is on (e.g., blood thinners, etc.); a condition of the patient (e.g., diabetes, cancer, etc.); or a different type of criteria.

For example, in the example of the heart failure exacerbation model, if a class of diabetes medication taken by a plurality of patients (e.g., more than the threshold number of correlated feedback records) causes the BP, HR, RR, and SpO2 to be outside expected ranges for these parameters, the additional criteria of having diabetes and/or taking the class of diabetes medication taken may be extracted from the standardized, predefined description and added to the alert generation model. The additional criteria may be added to the alert generation model by adding one or more additional rows to heart failure exacerbation model 600. As a result of including the additional criterion, a first accuracy of the alert generation model at generating alerts for non-diabetic-medication-taking patients may not be affected, and an accuracy of the alert generation model at generating alerts for diabetic-medication-taking patients may be increased.

In some cases, the inclusion of an additional rule or criteria in the alert generation model may spawn a new alert generation model, whereby the new alert generation model may be included in the patient monitoring system. For example, feedback records on an alert generation model may indicate that diabetic patients may be subject to different alert thresholds for generating an alert than non-diabetic patients. Based on the feedback records, a new criterion may be added to the alert generation model, where the new criterion includes an indication of whether the patient is a diabetic. As a result of adding the new criterion, a second alert generation model may be created, where the first alert generation model may be applied to patients who are not diabetics (e.g., where a new rule specifies that the patient is not a diabetic), and the second alert generation model may be applied to patients who are diabetics (e.g., where the new rule specifies that the patient is a diabetic). In this way, an accuracy of the patient monitoring system may be increased by evolving more specific and customized models, and adding new models where relevant.

At 426, method 400 includes calculating new alert thresholds for the data parameters included in each criterion of the alert generation model. In various embodiments, the new alert thresholds for each data parameter may be an average of the measured parameter values recorded for each patient of the stored feedback records classified as a false alert. The new alert thresholds may be generated based on data from the first set of patients, and applied to the second set of patients (e.g., the similar patients from the EMR) based on the additional criteria.

For example, with respect to the heart failure exacerbation model of FIG. 6A, the BP values measured from each patient at the time when the respective alert was generated in each feedback record may be averaged, and the average BP value may be used as the new alert threshold for BP for the set of similar patients; the HR values measured from each patient at the time when the respective alert was generated in each feedback record may be averaged, and the average HR value may be used as the new alert threshold for HR for the set of similar patients; the RR values measured from each patient at the time when the respective alert was generated in each feedback record may be averaged, and the average RR value may be used as the new alert threshold for RR for the set of similar patients; and so on. In this way, the alert thresholds may be adjusted from a first set of parameter values that are appropriate for a general population of patients, to a second set of parameter values that are more accurate for patients of the new category.

In other embodiments, a minimum parameter value of the feedback records may be used rather than an average parameter value, or a maximum parameter value, or a desired parameter value may be calculated based on a different formula. For example, using the minimum parameter values may result in a more cautious generation of alerts than using the average parameter values.

At 428, method 400 includes updating the rules-based alert generation model with the additional criteria and the new alert thresholds, and at 430, method 400 includes using the updated rules-based alert generation model to generate alerts for the similar patients (e.g., the second set of patients). In some embodiments, the updated rules-based alert generation model may be used to generate alerts for all patients, where the new alert thresholds are applied to patients meeting the added criteria, and not to patients not meeting the added criteria. In other embodiments, the updated rules-based alert generation model may be used as a separate model, to generate alerts only for patients meeting the added criteria.

In other words, the first set of patients may be identified and selected from feedback records indicating a false alert, where the first set of patients share a same standardized, predefined description of the false alert; and the second set of patients may be selected from a general population of patients using the patient monitoring system, where the second set of patients share one or more similar or defining characteristics with the first set of patients (e.g., as determined by a statistical technique such as a clustering model). The alert generation model may be updated to include the added criterion or criteria, and new alert thresholds may be generated based on patient data of the first set of patients. The alert generation model may then be applied to generate alerts for the second set of patients (which may include the first set of patients).

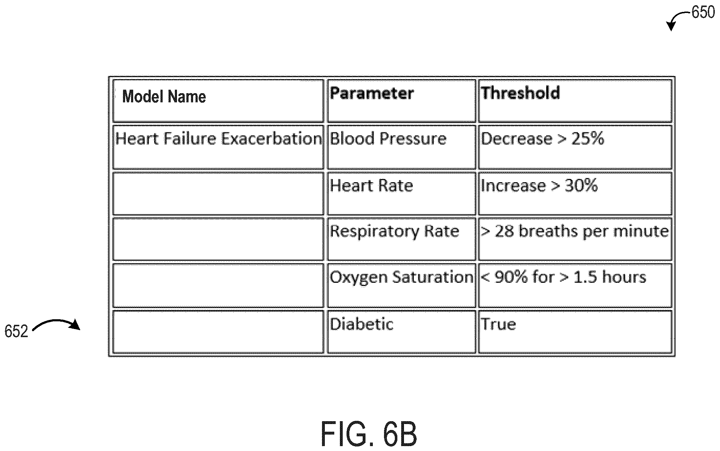

As an example of how method 400 could be used, FIG. 6B shows a second exemplary heart failure exacerbation model 650, where heart failure exacerbation model 650 may be a new alert generation model derived from heart failure exacerbation model 600 of FIG. 6A, in accordance with method 400 described above. That is, heart failure exacerbation model 650 may be a version of heart failure exacerbation model 600 applicable specifically to diabetic patients. Heart failure exacerbation model 650 includes the criteria used to generate an alert in heart failure exacerbation model 600, namely, BP, HR, RR, and SpO2. However, a fifth criterion 652 has been added, specifying that the patient is diabetic. Additionally, in heart failure exacerbation model 650, the BP alert threshold has been changed to reflect a decrease by greater than 25%, the HR alert threshold has been changed to reflect an increase by greater than 30%, the RR alert threshold has been changed to reflect a rate of more than 28 breaths per minute, and the SpO2 alert threshold has been changed to indicate a level less than 90% for more than 1.5 hours, in contrast to the alert thresholds of 20%, 25%, 24 breaths per minute, and 90% for more than 1 hour, respectively, of heart failure exacerbation model 600. Thus, heart failure exacerbation model 650 may be a more suitable alert generation model to apply to diabetic patients, while heart failure exacerbation model 600 may be a more suitable alert generation model to apply to non-diabetic patients.

In some embodiments, the updated alert generation model may be validated with actual patient data prior to being implemented or during implementation. That is, an original or current alert generation model may be used to generate an alert, and the updated generation model may be used to confirm the alert or compare results with the original or current alert generation model.

Thus, the disclosed systems and methods facilitate a closed-loop communication between caregivers, clinicians, and the patient monitoring system itself, where feedback from caregivers and clinicians could be incorporated into real-time data analysis, alert thresholds, and model updates, thereby ensuring that an accuracy and precision of alert generation models are continuously increased. Specifically, the patient monitoring system provided herein allows a caregiver to provide feedback for further training and/or updating of alert generation models more rapidly and efficiently than in an alternative patient monitoring systems. As a result, an accuracy and timeliness of patient monitoring may be increased, generating faster response times to changes in patient status, which may improve patient care and reduce costs for the patient and for a healthcare system. The technical effect of using the feedback collection systems and methods described herein to refine alert generation models used by a patient monitoring system is that an accuracy of the alert generation models may be increased.

The disclosure also provides support for a method for a patient monitoring system, the method comprising: receiving time-series patient data in real time from a patient monitor coupled to a patient, detecting, via an artificial intelligence (AI) model of the patient monitoring system detecting a deviation in the time-series patient data from expected time-series patient data, and in response to detecting the deviation, displaying an alert on the patient monitor, the alert including a graphical user interface (GUI) for receiving a feedback record regarding the alert from a user of the patient monitoring system: receiving a plurality of feedback records regarding alerts generated for a respective plurality of patients, via the GUI, and for each feedback record of the plurality of feedback records including a false alert, generating a standardized, predefined description of the false alert, determining that a number of feedback records having a same standardized, predefined description is greater than a threshold number, and in response: identifying a first set of patients having the same standardized, predefined description, identifying a second set of patients in an electronic medical record (EMR) database that have similar patient data and/or characteristics as the first set of patients, using a clustering model, the second set of patients including the first set of patients, updating the AI model based on patient data of the first set of patients, and using the updated AI model to generate alerts for the second set of patients. In a first example of the method, the AI model is a machine-learning (ML) model trained on training pair data using supervised learning, and updating the AI model based on patient data of the second set of patients further comprises generating a labeled dataset including patient data of the second set of patients and retraining the AI model on the labeled dataset. In a second example of the method, optionally including the first example, a training pair of the labeled dataset includes patient data of a single patient corresponding to a single feedback record, the patient data including vital sign measurements extracted from the single feedback record as input data, and an encoding of a false alert included in the feedback record as ground truth data. In a third example of the method, optionally including one or both of the first and second examples, the AI model is a rules-based model, and updating the AI model based on patient data of the second set of patients further comprises: calculating an amount of correlated feedback records, the correlated feedback records including the same standardized, predefined description of a false alert, in response to the amount of feedback records being greater than a threshold amount, updating the AI model based on patient data of the second set of patients, and in response to the amount of feedback records not being greater than the threshold amount, not updating the AI model based on patient data of the second set of patients. In a fourth example of the method, optionally including one or more or each of the first through third examples, updating the AI model based on patient data of the second set of patients further comprises extracting one or more additional criteria to include in the AI model from the standardized, predefined description of the correlated feedback records, and adding the one or more additional criteria to the AI model. In a fifth example of the method, optionally including one or more or each of the first through fourth examples, updating the AI model based on patient data of the second set of patients further comprises calculating new alert thresholds for patient data parameters of the AI model. In a sixth example of the method, optionally including one or more or each of the first through fifth examples, a new alert threshold for a patient data parameter of the AI model is an average of measured parameter values recorded for each patient of the first set of patients. In a seventh example of the method, optionally including one or more or each of the first through sixth examples, a new alert threshold for a patient data parameter of the AI model is a minimum of measured parameter values recorded for each patient of the first set of patients. In a eighth example of the method, optionally including one or more or each of the first through seventh examples, updating the AI model based on patient data of the second set of patients further comprises generating a new alert generation model, the new alert generation model including the one or more additional criteria. In a ninth example of the method, optionally including one or more or each of the first through eighth examples, the feedback record is received via a feedback collection panel of the GUI, the feedback collection panel including a plurality of numbered code buttons that allow the user to provide predefined feedback messages regarding the alert in accordance with a respective plurality of predefined codes stored in a memory of the patient monitoring system. In a tenth example of the method, optionally including one or more or each of the first through ninth examples, the feedback collection panel further includes a comments field configured to receive a textual feedback message regarding the alert. In a eleventh example of the method, optionally including one or more or each of the first through tenth examples, generating the standardized, predefined description of the false alert further comprises: identifying a keyword included in the textual feedback message using one or more lookup tables stored in the memory, extracting the keyword from the textual feedback, and mapping the extracted keyword and a predefined feedback message to a standardized, predefined description of the false alert in a reference database of the patient monitoring system. In a twelfth example of the method, optionally including one or more or each of the first through eleventh examples, the textual feedback message is generated from a voice recording of a feedback message recorded using a control element of the feedback collection panel. In a thirteenth example of the method, optionally including one or more or each of the first through twelfth examples, the feedback collection panel is displayed based on historical patterns of a patient data parameter included in the AI model.

The disclosure also provides support for a patient monitoring system, comprising: a patient monitor coupled to a patient, an alert generation model configured to display an alert on the patient monitor in response to detecting a deviation in time-series patient data transmitted from the patient monitor, the alert including a graphical user interface (GUI) for receiving feedback regarding the alert from a user of the patient monitoring system, a processor, and a memory storing instructions that when executed, cause the processor to: in response to a plurality of feedback records received via the GUI exceeding a threshold number of feedback records: for each feedback record of the plurality of feedback records that indicates a false alert, generate a standardized, predefined description of the false alert, in response to a number of feedback records having a same standardized, predefined description being greater than a threshold number: identify a first set of patients of the number of feedback records having the same standardized, predefined description, identify a second set of patients in an electronic medical record (EMR) database coupled to the patient monitoring system that are similar to the first set of patients, using a clustering model, the second set of patients including the first set of patients, update the alert generation model based on patient data of the first set of patients, and use the updated alert generation model to generate alerts for the second set of patients. In a first example of the system, further instructions are stored in the memory that when executed, cause the processor to retrain the alert generation model on a labeled dataset of training pairs, where a training pair of the labeled dataset includes vital sign measurements extracted from a feedback record as input data, and an encoding of a false alert included in the feedback record as ground truth data. In a second example of the system, optionally including the first example, further instructions are stored in the memory that when executed, cause the processor to: calculate an amount of correlated feedback records, the correlated feedback records having the same standardized, predefined description of a false alert, extract an additional criteria to include in the alert generation model from the standardized, predefined description of the correlated feedback records, calculate a new alert threshold for a patient data parameter of the alert generation model based on the additional criteria, the new alert threshold an average of measured parameter values recorded for each patient of the first set of patients, and update the alert generation model with the additional criteria and new alert threshold. In a third example of the system, optionally including one or both of the first and second examples, the feedback is received via a feedback collection panel of the GUI, the feedback collection panel including a plurality of numbered code buttons that allow the user to provide predefined feedback messages regarding the alert in accordance with a respective plurality of predefined codes stored in a memory of the patient monitoring system, and a comments field that allows the user to enter in a textual feedback message regarding the alert. In a fourth example of the system, optionally including one or more or each of the first through third examples, further instructions are stored in the memory that when executed, cause the processor to: identify a keyword included in a textual feedback message using one or more lookup tables stored in the memory, extract the keyword from the textual feedback, and map the extracted keyword and a predefined feedback message to a standardized, predefined description of the false alert in a reference database of the patient monitoring system.

The disclosure also provides support for a method for a patient monitoring system, the method comprising: receiving a first plurality of feedback records from users of the patient monitoring system with respect to alerts generated for a respective plurality of patients by an AI model, extracting a second plurality of feedback records including an indication of a false alert from the first plurality of feedback records, for each feedback record of the second plurality of feedback records: retrieving a predefined feedback message from a first lookup table stored in a memory of the patient monitoring system, based on a code included in the feedback record, identifying a keyword included in a textual feedback message of the feedback record using a second lookup table stored in the memory of the patient monitoring system, and extracting the keyword from the textual feedback, mapping the extracted keyword and the predefined feedback message to a standardized, predefined description of the false alert stored in a reference database of the patient monitoring system, and including the standardized, predefined description in the feedback record, selecting a first set of patients of the second plurality of feedback records that have the same standardized, predefined description, identifying a second set of patients in an electronic medical record (EMR) database that have similar patient data and/or patient characteristics as the first set of patients, using a clustering model, extracting one or more additional criteria to include in the AI model from the standardized, predefined description of the correlated feedback records, and adding the one or more additional criteria to the AI model, calculating new alert thresholds for patient data parameters of the AI model, each new alert threshold an average of corresponding measured parameter values recorded for each patient of the first set of patients, and using the updated AI model to generate alerts for the second set of patients via the patient monitoring system.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A method (300, 400) for a patient monitoring system, the method comprising:
receiving time-series patient data in real time from a patient monitor coupled to a patient (306);
detecting, via an artificial intelligence (AI) model of the patient monitoring system detecting a deviation in the time-series patient data from expected time-series patient data, and in response to detecting the deviation, displaying an alert on the patient monitor, the alert including a graphical user interface (GUI) for receiving a feedback record regarding the alert from a user of the patient monitoring system (310):
receiving a plurality of feedback records regarding alerts generated for a respective plurality of patients, via the GUI (312); and
for each feedback record of the plurality of feedback records including a false alert, generating a standardized, predefined description of the false alert (404);
determining that a number of feedback records having a same standardized, predefined description is greater than a threshold number (420), and in response:
identifying a first set of patients having the same standardized, predefined description (410);
identifying a second set of patients in an electronic medical record (EMR) database that have similar patient data and/or characteristics as the first set of patients, using a clustering model (412), the second set of patients including the first set of patients;
updating the AI model based on patient data of the first set of patients (416, 418); and
using the updated AI model to generate alerts for the second set of patients (417, 430).

2. The method (300, 400) of claim 1, wherein the AI model is a machine-learning (ML) model trained on training pair data using supervised learning, and updating the AI model based on patient data of the second set of patients further comprises generating a labeled dataset including patient data of the second set of patients and retraining the AI model on the labeled dataset (416).

3. The method (300, 400) of claim 2, wherein a training pair of the labeled dataset includes patient data of a single patient corresponding to a single feedback record, the patient data including vital sign measurements extracted from the single feedback record as input data, and an encoding of a false alert included in the feedback record as ground truth data.

4. The method (300, 400) of claim 1, wherein the AI model is a rules-based model, and updating the AI model based on patient data of the second set of patients further comprises:
calculating an amount of correlated feedback records, the correlated feedback records including the same standardized, predefined description of a false alert (420);
in response to the amount of feedback records being greater than a threshold amount, updating the AI model based on patient data of the second set of patients; and
in response to the amount of feedback records not being greater than the threshold amount, not updating the AI model based on patient data of the second set of patients (422).

5. The method (300, 400) of claim 4, wherein updating the AI model based on patient data of the second set of patients further comprises extracting one or more additional criteria to include in the AI model from the standardized, predefined description of the correlated feedback records, and adding the one or more additional criteria to the AI model (424).

6. The method (300, 400) of claim 5, wherein updating the AI model based on patient data of the second set of patients further comprises calculating new alert thresholds for patient data parameters of the AI model (426).

7. The method (300, 400) of claim 6, wherein a new alert threshold for a patient data parameter of the AI model is an average of measured parameter values recorded for each patient of the first set of patients.

8. The method (300, 400) of claim 6, wherein a new alert threshold for a patient data parameter of the AI model is a minimum of measured parameter values recorded for each patient of the first set of patients.

9. The method (300, 400) of claim 5, wherein updating the AI model based on patient data of the second set of patients further comprises generating a new alert generation model, the new alert generation model including the one or more additional criteria.

10. The method (300, 400) of claim 1, wherein the feedback record is received via a feedback collection panel of the GUI, the feedback collection panel including a plurality of numbered code buttons that allow the user to provide predefined feedback messages regarding the alert in accordance with a respective plurality of predefined codes stored in a memory of the patient monitoring system.

11. The method (300, 400) of claim 10, wherein the feedback collection panel further includes a comments field configured to receive a textual feedback message regarding the alert.

12. The method (300, 400) of claim 11, wherein generating the standardized, predefined description of the false alert further comprises:
identifying a keyword included in the textual feedback message using one or more lookup tables stored in the memory;
extracting the keyword from the textual feedback; and
mapping the extracted keyword and a predefined feedback message to a standardized, predefined description of the false alert in a reference database of the patient monitoring system (408).

13. The method (300, 400) of claim 12, wherein the textual feedback message is generated from a voice recording of a feedback message recorded using a control element of the feedback collection panel.

14. The method (300, 400) of claim 10, wherein the feedback collection panel is displayed based on historical patterns of a patient data parameter included in the AI model.

15. A patient monitoring system (220), comprising:
a patient monitor (110) coupled to a patient (102);
an alert generation model (500, 600, 650) configured to display an alert on the patient monitor (110) in response to detecting a deviation in time-series patient data transmitted from the patient monitor (110), the alert including a graphical user interface (GUI) (700) for receiving feedback regarding the alert from a user of the patient monitoring system (220);
a processor (206), and a memory (204) storing instructions that when executed, cause the processor (206) to:
in response to a plurality of feedback records (900) received via the GUI (700) exceeding a threshold number of feedback records (900):
for each feedback record (900) of the plurality of feedback records (900) that indicates a false alert, generate a standardized, predefined description of the false alert;
in response to a number of feedback records (900) having a same standardized, predefined description being greater than a threshold number:
identify a first set of patients of the number of feedback records (900) having the same standardized, predefined description;
identify a second set of patients in an electronic medical record (EMR) database (222) coupled to the patient monitoring system (220) that are similar to the first set of patients, using a clustering model, the second set of patients including the first set of patients;
update the alert generation model (500, 600, 650) based on patient data of the first set of patients; and
use the updated alert generation model (500, 600, 650) to generate alerts for the second set of patients.
